# EUROPEAN PATENT APPLICATION

(11) **EP 1 938 837 A1**
(43) Date of publication of application: **02.07.2008**
(21) Application number: 06796870.1
(22) Date of filing: 28.08.2006
(51) Int. Cl.: A61K 45/00, A61K 31/137, A61K 31/195, A61K 31/216, A61K 31/472, A61P 27/02, A61P 43/00

(54) **PREVENTIVE OR THERAPEUTIC AGENT FOR DISEASE CAUSED BY DECREASE IN LACRIMAL FLUID**

(30) Priority: 29.08.2005 JP 2005247563
(71) Applicant: Kissei Pharmaceutical Co., Ltd., Matsumoto-shi Nagano 399-8710 (JP)
(72) Inventor: KOBAYASHI, Mamoru, Azumino-shi Nagano 399-8304 (JP); ASARI, Tetsuya, Azumino-shi Nagano 399-8304 (JP)
(74) Representative: Behnisch, Werner
(86) International application number: PCT/JP2006/316836
(87) International publication number: WO 2007/026630

(57) **Abstract**

The present invention provides pharmaceuticals for the prevention or treatment of diseases associated with decrease in tear. That is, the present invention provides pharmaceuticals for the prevention or treatment of diseases associated with decrease in tear such as dry eye, dry disorders of cornea and conjunctiva, disorders of the keratoconjunctival epithelium, syndrome with decrease in tear secretion, xerophthalmia, dry eye due to aging, ophthalmopathy in Stevens-Johnson syndrome, ophthalmopathy in Sjögren's syndrome, keratoconjunctival ulcer, dryness in wearing of contact lens or the like, which comprises a β₃ adrenoceptor stimulant. The present invention also provides a combination pharmaceutical comprising a β₃ adrenoceptor stimulant and a β₂ adrenoceptor stimulant.

## Description

### Field of the invention

The present invention relates to a pharmaceutical useful for the prevention or treatment of a disease associated with decrease in tear.

More specifically, the present invention relates to a pharmaceutical for the prevention or treatment of diseases associated with decrease in tear, which comprises a β₃ adrenoceptor (hereinafter referred to as "β₃ AR") stimulant. The present invention also relates to a combination pharmaceutical additionally comprising a β₂ adrenoceptor (hereinafter referred to as "β₂ AR") stimulant.

### Background Art

A typical disease associated with decrease in tear is dry eye. By the Dry Eye Study Group, dry eye is defined as disorders of the keratoconjunctival epithelium caused by qualitative or quantitative abnormality of tear (lacrimal layer) and diagnostic criteria of dry eye based on examination of qualitative and quantitative abnormalities of the tear (ocular layer) and disorders of the keratoconjunctival epithelium have been proposed (for example, see Non-patent reference 1). The diagnostic criteria have been reinvestigated every 10 years, and the 1995 criteria are currently used widely in Japan. Overseas, the NIH diagnostic criteria (Lemp et al. 1995) are used in general. As major subjective symptoms of dry eye, dryness, pain, itching of eyes, blurring of vision due to disorders of the keratoconjunctival epithelium, severe vision disorders and the like are known.

In addition to dry eye, the following diseases can be considered to be syndromes with similar symptoms that are associated with decrease in tear: dry disorders of cornea and conjunctiva, disorders of the keratoconjunctival epithelium, syndrome with decrease in tear secretion, xerophthalmia, dry eye due to aging, ophthalmopathy in Stevens-Johnson syndrome, ophthalmopathy in Sjögren's syndrome, keratoconjunctival ulcer, oligodacrya, keratoconjunctivitis sicca, ocular pemphigus, blepharitis marginalis, insufficient occlusion of eye lids, sensory neuroparalysis, allergic conjunctivitis, and dryness post-viral conjunctivitis, post-cataract surgery, in wearing of contact lens or in operation of visual display terminal (VDT).

The principal treatment of these diseases associated with decrease in tear is pharmacotherapy using ophthalmic preparations, although surgeries such as plug in the lacrimal puncta and-punctual occlusion are also performed. Principally employed pharmacotherapy includes artificial tears for the purpose of increasing tear and eye drops of sodium hyaluronate for the purpose of stabilizing the tear on the keratoconjunctival epithelium. However, whereas the aqueous layer, lipid layer and mucous layer, which form the lacrimal layer, are said to be important to maintain the healthy keratoconjunctival surface, drugs used currently are not sufficiently effective. When it is caused by allergy or inflammation, steroidal and anti-allergic eye drops are used. However, adverse reactions such as increase in intraocular pressure induced by prolonged administration of steroids are sometimes problematic. The number of patients continues to increase, as the environment of patients changes like increase in operations with staring at OA instruments, an air pollution and allergy. Thus, early development of an effective therapeutic agent is desired (for example, see Non-patent reference 2).

There are two factors in decrease in tear. One is decrease in tear secretion and the other is increase in evaporation and excretion of tear. Tear secretion mainly occurs in two ways. One is reflex secretion induced by stimulation to the region controlled by the trigeminal nerve (cornea, conjunctiva, skin, nose, emotion and the like.). The other is basic secretion, which protects the keratoconjunctival surface. In nerve pathways that facilitate tear secretion, there are three of the trigeminal, parasympathetic and sympathetic nerves (see Non-patent reference 3). Although reflex secretion is said to be provided chiefly by the main lacrimal gland, other secretory glands may be playing roles (see Non-patent reference 4). It is reported that the main lacrimal gland is controlled by the sympathetic and parasympathetic nerves or neuropeptides such as neuropeptide Y. As tear secretion is suppressed by β-adrenoceptor blockers such as propranolol, it is thought that β₁ AR or β₂ AR subtype plays a role in tear secretion (see Non-patent reference 5). On the other hand, lipid secretion, which prevents the evaporation of tear, originates in the meibomian glands, Zeis glands and Moll glands (see Non-patent reference 4), and is controlled by the sympathetic and parasympathetic nerves and neuropeptides such as substance P. In addition, it has been elucidated that glycoproteins such as mucin that forms the mucous layer are secreted from goblet cells and mucous cells in the lacrimal gland (see Non-patent reference 6). However, the detailed mechanism of the secretion remains unclear. It is said that hypofunction of these lipid and mucous secretion causes the breakdown of the lacrimal three-layer structure of the keratoconjunctiva and plays an important role in dry eye as a result.

Tear supplied by the lacrimal gland and keratoconjunctiva contains bioactive substances such as various electrolytes, proteins and vitamin A. Among proteins secreted in tear, mucin is known as a glycoprotein originating from the keratoconjunctiva and is thought to protect principally the eyeball. As proteins originating from the lacrimal gland, mucin, lactoferrin, lipocaine, IgA, complement, fibronectin, EGF (epithelial growth factor), HGF (hepatocellular growth factor), TGF (transforming growth factor)β₁, TGFβ₂, various cytokines, amylase, SOD (superoxide dismutase), lysozyme and the like are known. These proteins are thought to be in charge of prevention of evaporation of moisture, antibacterial action and nutrition supply to the ophthalmic tissues and the like. Development of a drug that repairs injured sites of the keratoconjunctival epithelium in dry eye and other diseases and prevents the exacerbation by facilitating the secretion of tear which contains these proteins are desired.

In these years, β₃ AR subtype has been reported as a subtype of β-adrenoceptor of sympathetic nerve system. However, the existence or physiology of this subtype in the visual organ has not ever been reported. On the other hand, it has been reported that a β₃ AR stimulant is useful for the prevention or treatment of obesity, hyperglycemia, a disease caused by intestinal tract hypermotility, pollakiuria or urinary incontinence, depression, a disease caused by biliary calculus or biliary tract hypermotility or the like (see Patent references 1 and 2). However, it has not ever been reported or suggested that a β₃ AR stimulant is useful for the prevention or treatment of a disease associated with decrease in tear such as dry eye.

It is known that a β₂ AR stimulant exerts an inhibitory effect against smooth muscle contraction and is useful as an agent for the treatment of bronchial asthma, threatened abortion or premature labor or the like (for example, see Non-patent reference 7). In addition, it has been reported that a β₂ AR stimulant which is β₂-selective compared to its β₁ AR stimulating activity has facilitating effects of tear secretion and/or protein secretion in tear, is useful for the prevention and treatment of dry ophthalmopathy or the like and has an advantage in that it has a less cardioactivity than a β AR stimulant having a β₁ AR stimulating activity (Patent reference 3). Nothing, however, has been described or suggested about an effect of a β₃ AR stimulant or a combination effect of a β₂ AR stimulant and a β₃ AR stimulant on tear secretion in these references.

As a β₃ AR stimulant having a β₂ AR stimulating activity, a kind of aminoethylphenoxyacetic acid derivatives are known and have been reported to be useful as an agent for relieving pain and promoting the removal of calculi in urolithiasis (for example, see Patent reference 4). However, the reference does neither describe nor suggest these aminoethylphenoxyacetic acid derivatives are useful for the diseases associated with decrease in tear.

[Patent reference 1] International publication No. WO00-02846 pamphlet
[Patent reference 2] International publication No. WO2004-072016 pamphlet
[Patent reference 3] International publication No. WO01-41806 pamphlet
[Patent reference 4] International publication No. WO99-05090 pamphlet
[Non-patent reference 1] Jun Shimazaki et al., Ganka (Ophthalmology), 1995, Vol.37, pp.765-770
[Non-patent reference 2] Edited by Kazuo Tsubota, Dry eye clinic, Igakusyoin, 2000, pp.43-53
[Non-patent reference 3] Edited by Yoshihisa Oguchi et al., Ocular Surface no Shindan to Chiryo (Diagnosis and Treatment of Ocular Surface), Medical Aoi Publication, 1993, pp.15-30
[Non-patent reference 4] Lemp M.A. et al., The lacrimal apparatus. Adler's physiology of the eye, Edit.9, Mosby Year Book company, 1992, pp.18-28
[Non-patent reference 5] Petounis A.D. et al., Int. Ophthalm., 1989, Vol.13, pp.75-80
[Non-patent reference 6] Sullivan D.A. et al., Lacrimal grand, tear film and dry eye syndromes, Plenum Press company, 1994, pp.1-9
[Non-patent reference 7] Edited by Chikako Tanaka et al., NEW Yakurigaku (New pharmacology), Nankodo, 2002, pp.227-236

### Disclosure of the invention

### Problem that the invention aims to solve

The purpose of the present invention is to provide a pharmaceutical for the prevention or treatment of a disease associated with decrease in tear.

### Means to solve the problem

As the result of earnest research on the above-mentioned problem, the present inventors newly found that β₃ AR exists in main and accessory lacrimal glands and mucous secretion cells and β₃ AR stimulants increase the quantities of tear secretion and protein secretion in tear. In addition, surprisingly, the inventors also found that a combination administration of a β₃ AR stimulant and a β₂ AR stimulant has a more excellent effect than a selective β₂ AR stimulant, and thereby forming the basis of the present invention.

That is, the present invention relates to:
[1] a pharmaceutical for the prevention or treatment of a disease associated with decrease in tear, the facilitation of tear secretion or the facilitation of protein secretion in tear which comprises a β₃ AR stimulant, a β₃ AR stimulant having a β₂ AR stimulating activity or a combination pharmaceutical comprising a β₃ AR stimulant and a β₂ AR stimulant;
[2] a pharmaceutical as described in the above [1] wherein the disease associated with decrease in tear are one or more diseases selected from the group consisting of dry eye, dry disorders of cornea and conjunctiva, disorders of the keratoconjunctival epithelium, syndrome with decrease in tear secretion, xerophthalmia, dry eye due to aging, ophthalmopathy in Stevens-Johnson syndrome, ophthalmopathy in Sjögren's syndrome, keratoconjunctival ulcer, oligodacrya, keratoconjunctivitis sicca, ocular pemphigus, blepharitis marginalis, insufficient occlusion of eye lids, sensory neuroparalysis, allergic conjunctivitis, and dryness post-viral conjunctivitis, post-cataract surgery, in wearing of contact lens or in operation of visual display terminal (VDT);
[3] a pharmaceutical as described in the above [1] or [2] wherein the dosage form is an oral formulation or a parenteral formulation such as an eye drop; and the like.

### Effect of the invention

Pharmaceuticals comprising a β₃ AR stimulant of the present invention exert facilitating effects of tear secretion and protein secretion in tear and are useful for the prevention or treatment of diseases associated with decrease in tear such as dry eye or the like.

### Brief description of the drawing

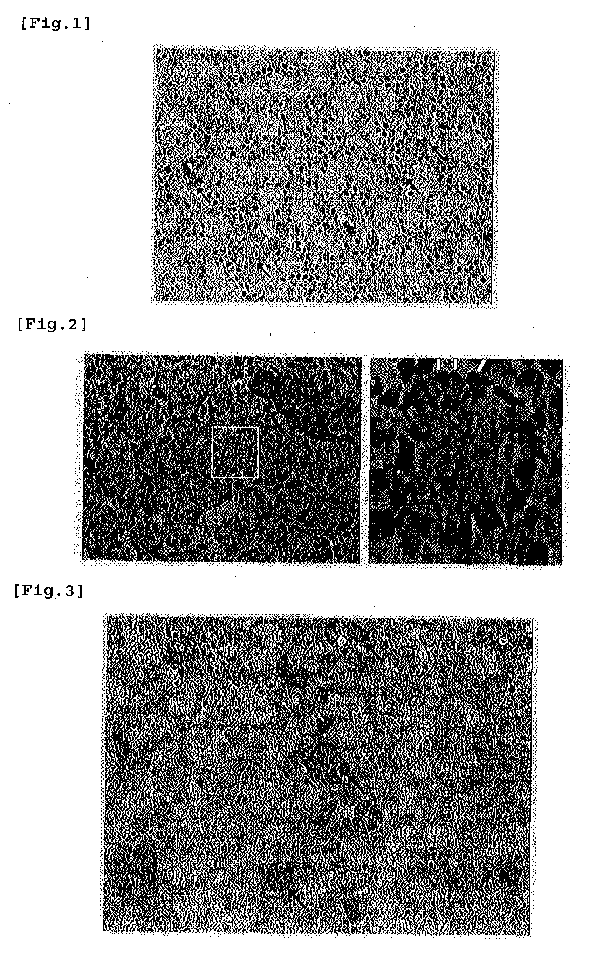

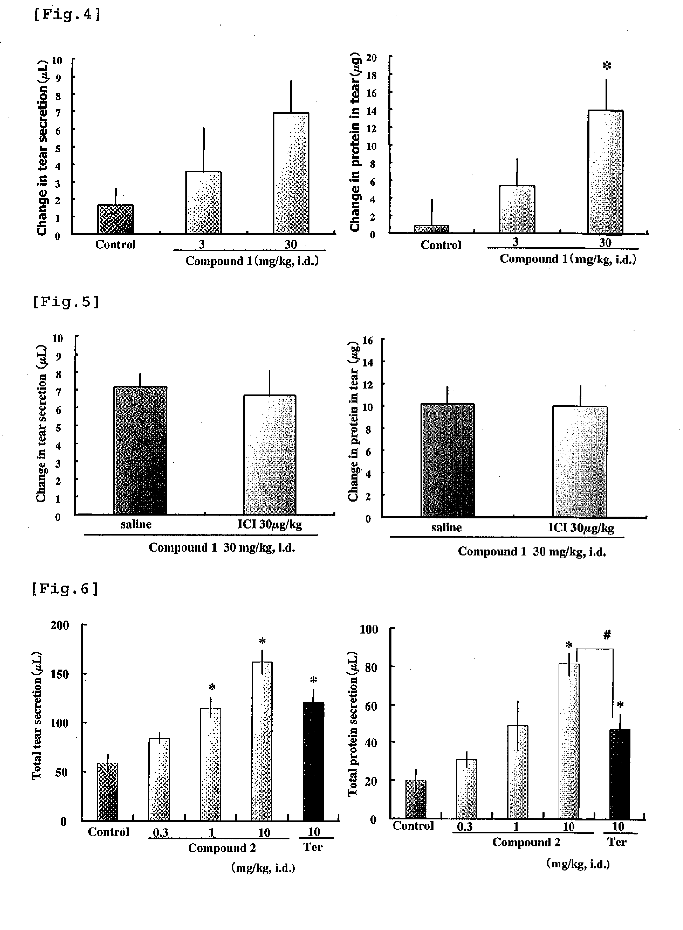

[Figure 1]
   Results of immunostaining of the main lacrimal gland extirpated from rabbits are shown. In the figure, stained portions (indicated by arrows) indicate the distribution of β₃ AR.
[Figure 2]
   Results of immunostaining of human lacrimal glands are shown. In the figure, stained portions indicate the distribution of β₃ AR. The figure on the right shows an enlargement of the framed area in the figure on the left, and arrows indicate stained portions.
[Figure 3]
   Results of PAS staining of the main lacrimal gland extirpated from rabbits are shown. In the figure, stained portions (indicated by arrows) indicate the distribution of glycoproteins (mucin and the like).
[Figure 4]
   Actions of Compound 1(administered into the duodenum) on the tear secretion in rabbits are shown. The figure on the left shows changes in the quantity of tear secretion(µL). The figure on the right shows changes in the quantity of protein in tear(µg). In each figure, the axis of abscissas indicates drug administration groups, which are, from left, Control: Control (vehicle) group, Compound 1 (3 mg/kg) group and Compound 1 (30 mg/kg) group. The changes in the quantity on the axis of ordinates indicate differences between the quantity measured for 5 min at 20 min after drug administration and the quantity measured for 5 min before drug administration. Each value is mean ±SD of 4 animals. The "*" indicates p<0.05 (significant difference compared to Control group in Dunnett test).
[Figure 5]
   Actions of Compound 1(administered into the duodenum) on the tear secretion in rabbits are shown. The figure on the left shows changes in the quantity of tear secretion (µL). The figure on the right shows changes in the quantity of protein in tear (µg). In each figure, the axis of abscissas indicates treatments given 5 min before administration of Compound 1, which are, from left, Saline: physiological saline administration group and ICI:ICI-118551, a selective β₂ AR inhibitor (30µg/kg) treatment group. The changes in the quantity on the axis of ordinates have the same meanings as defined in Figure 3. Each value is mean ±SD of 5 animals.
[Figure 6]
   Actions of Compound 2 or terbutaline sulfate (administered into the duodenum) on the tear secretion in rabbits are shown. The figure on the left shows the total quantity of tear secretion (µL), and the figure on the right shows the total quantity of protein in tear (µg). In each figure, the axis of abscissas indicates drug administration groups, which are, from left, Control: Control (vehicle) group, Compound 2 (0.3 mg/kg) group, Compound 2 (1 mg/kg) group, Compound 2 (10 mg/kg) group and Ter: Terbutaline sulfate (10 mg/kg) group. The axis of ordinates indicates the total quantity measured for 60 min after administration of test drugs. Each values is mean ±SD of 4 animals. The "*" indicates p<0.05 (significant difference compared to Control group in Dunnett test or t-test), and the "#" indicates p<0.05 (significant difference compared to Terbutaline sulfate group in t-test).
[Figure 7]
   Actions of Compound 2 or terbutaline sulfate (administered into the duodenum) on the tear secretion in rats are shown. The figure on the left shows the total quantity of tear secretion (µL), and the figure on the right shows the total quantity of protein in tear (µg). In each figure, the axis of abscissas has the same meaning as defined in Figure 5, and the axis of ordinates indicates the total quantity measured for 60 min after administration of a test drug. Each value is mean ±SD of 9 to 10 animals. The "*" indicates p<0.05 (significant difference compared to Control group in Dunnett test), "N. S." means no significant difference (compared to Terbutaline sulfate group in t-test), and the "#" has the same meaning as defined in Figure 6.
[Figure 8]
   Actions of Compound 2 (0.1% solution, 50µL) in single instillation to rabbit eyes on tear secretion are shown. The axis of abscissas expresses time (the time after administration) (minute) when measurement was performed in, from left, Control: Control (vehicle) group and Compound 2 (0.1% solution) group at each time. The axis of ordinates indicates changes in the quantity of tear secretion (µL) compared to that 5 min before administration. Each value is mean ±SD of 4 animals. The "*" indicates p<0.05 (significant difference compared to Control group in t-test).
[Figure 9]
   Actions of Compound 2 or terbutaline sulfate (ocular instillation) on mucin secretion in the rabbit conjunctiva are shown. The axis of abscissas indicates drug administration groups, which are, from left, Control: Control (vehicle) group, Compound 2 (0.1% solution) group and Ter: Terbutaline sulfate (0.1% solution) group. The axis of ordinates indicates the count of PAS positive goblet cells in a certain visual field. Each value is mean ±SD of 4animals. The "*" indicates p<0.05 (significant difference compared to Control group in t-test).

### Best mode to operate the invention

As a β₃ AR stimulant used in the present invention, a compound having a stronger β₃ AR stimulating activity than its β₁ AR stimulating activity, especially, 10 times or stronger one is preferable, and 100 times or stronger one is more preferable. The activity stimulating each receptor can be determined by methods such as known binding studies, functional studies using extracted organs or the like (for example, as described in Patent reference 4). As concrete compounds, for example, compounds described in Patent reference 1, 2 or 4, BRL37344, ZD2079, CGP12177, CL316243, L-796568, Ro40-2148, ICID7114, YM-178, solabegron and the like can be illustrated.

As preferable compounds in β₃ AR stimulants, for example, compounds described in Patent reference 1:
2-[2-bromo-4-[2-[[(1S,2R)-2-hydroxy-2-(4-hydroxyphenyl)-1-methylethyl]amino]ethyl]phenoxy]acetic acid,
ethyl 2-[2-bromo-4-[2-[[(1S,2R)-2-hydroxy-2-(4-hydroxyphenyl)-1-methylethyl]amino]ethyl]phenoxy]acetate,
2-[2-chloro-4-[2-[[(1S,2R)-2-hydroxy-2-(4-hydroxyphenyl)-1-methylethyl]amino]ethyl]phenoxy]acetic acid,
ethyl 2-[2-chloro-4-[2-[[(1S,2R)-2-hydroxy-2-(4-hydroxyphenyl)-1-methylethyl]amino]ethyl]phenoxy]acetate,
2-[2,5-dichloro-4-[2-[[(1S,2R)-2-hydroxy-2-(4-hydroxyphenyl)-1-methylethyl]amino]ethyl]phenoxy]acetic acid,
ethyl 2-[2,5-dichloro-4-[2-[[(1S,2R)-2-hydroxy-2-(4-hydroxyphenyl)-1-methylethyl]amino]ethyl]phenoxy]acetate,
2-[4-[2-[[(1S,2R)-2-hydroxy-2-(4-hydroxyphenyl)-1-methylethyl]amino]ethyl]-2,5-dimethylphenoxy]acetic acid,
ethyl 2-[4-[2-[[(1S,2R)-2-hydroxy-2-(4-hydroxyphenyl)-1-methylethyl]amino]ethyl]-2,5-dimethylphenoxy]-acetate (hereinafter referred to as Compound 1),
2-[2-hydroxy-4-[2-[[(1S,2R)-2-hydroxy-2-(4-hydroxyphenyl)-1-methylethyl]amino]ethyl]phenoxy]acetic acid,
ethyl 2-[2-hydroxy-4-[2-[[(1S,2R)-2-hydroxy-2-(4-hydroxyphenyl)-1-methylethyl]amino]ethyl]phenoxy]acetate, or a pharmaceutically acceptable salt thereof;

compounds described in Patent reference 2:
4'-{2-[(1S,2R)-2-hydroxy-2-(4-hydroxyphenyl)-1-methyl ethylamino]ethoxy}-2,3',5'-trimethylbiphenyl-4-carboxylic acid,
4'-{2-[(1S,2R)-2-hydroxy-2-(4-hydroxyphenyl)-1-methyl ethylamino]ethoxy}-3-isopropyl-3',5'-dimethylbiphenyl-4-carboxylic acid,
(3-acetyl-4'-{2-[(1S,2R)-2-hydroxy-2-(4-hydroxyphenyl)-1-methylethylamino]ethoxy}-3',5'-dimethylbiphenyl-4-yloxy)acetic acid,
4'-{2-[(1R,2S)-2-hydroxy-2-(4-hydroxyphenyl)-1-methylethylamino]ethoxy}-2,2'-dimethylbiphenyl-4-caboxylic acid,
2-ethyl-4'-{2-[(1S,2R)-2-hydroxy-2-(4-hydroxyphenyl)-1-methylethylamino]ethoxy}-2'-methylbiphenyl-4-carboxylic acid,
4'-{2-[(1S,2R)-2-hydroxy-2-(4-hydroxyphenyl)-1-methylethylamino]ethoxy}-2-isopropyl-2'-methylbiphenyl-4-carboxylic acid,
4'-{2-[(1S,2R)-2-hydroxy-2-(4-hydroxyphenyl)-1-methylethylamino]ethoxy}-2'-methyl-2-propylbiphenyl-4-carboxylic acid,
4'-{2-[(1R,2S)-2-hydroxy-2-(4-hydroxyphenyl)-1-methylethylamino]ethoxy}-2-methoxy-3',5'-dimethylbiphenyl-4-carboxylic acid,
4'-{2-[(1R,2S)-2-hydroxy-2-(4-hydroxyphenyl)-1-methylethylamino]ethoxy}-3',5'-dimethyl-2-propylbiphenyl-4-carboxylic acid,
2-ethyl-4'-{2-[(1R,2S)-2-hydroxy-2-(4-hydroxyphenyl)-1-methylethylamino]ethoxy}-3'-methylbiphenyl-4-carboxylic acid,
4'-{2-[(1R,2S)-2-hydroxy-2-(4-hydroxyphenyl)-1-methylethylamino]ethoxy}-3'-methyl-2-propylbiphenyl-4-carboxylic acid,
3-cyclopentyl-4'-{2-[(1R,2S)-2-hydroxy-2-(4-hydroxyphenyl)-1-methylethylamino]ethoxy}-3'-methylbiphenyl-4-carboxylic acid,
2-ethyl-3'-fluoro-4'-{2-[(1R,2S)-2-hydroxy-2-(4-hydroxyphenyl)-1-methylethylamino]ethoxy}biphenyl-4-carboxylic acid,
3'-fluoro-4'-{2-[(1R,2S)-2-hydroxy-2-(4-hydroxyphenyl)-1-methylethylamino]ethoxy}-2-isopropylbiphenyl-4-carboxylic acid,
3'-fluoro-4'-{2-[(1R,2S)-2-hydroxy-2-(4-hydroxyphenyl)-1-methylethylamino]ethoxy}-2-propylbiphenyl-4-carboxylic acid,
(4'-{2-[(1S,2R)-2-hydroxy-2-(4-hydroxyphenyl)-1-methylethylamino]ethoxy}-2,3',5'-trimethylbiphenyl-4-yloxy)acetic acid,
3-hyroxy-4'-{2-[(1S,2R)-2-hydroxy-2-(4-hydroxyphenyl)-1-methylethylamino]ethoxy}-3',5'-dimethylbiphenyl-4-carboxylic acid,
4'-{2-[(1R,2S)-2-hydroxy-2-(4-hydroxyphenyl)-1-methylethylamino]ethoxy}-3',5'-dzmethyl-3-(p-tolyloxy)-biphenyl-4-carboxylic acid,
3-(4-chlorophenoxy)-4'-{2-[(1R,2S)-2-hydroxy-2-(4-hydroxyphenyl)-1-methylethylamino]ethoxy}-3',5'-dimethylbiphenyl-4-carboxylic acid,
3-(4-fluorophenoxy)-4'-{2-[(1R,2S)-2-hydroxy-2-(4-hydroxyphenyl)-1-methylethylamino]ethoxy}-3',5'-dimethylbiphenyl-4-carboxylic acid,
4'-{2-[(1R,2S)-2-hydroxy-2-(4-hydroxyphenyl)-1-methylethylamino]ethoxy}-3-(4-methoxyphenoxy)-3',5'-dimethylbiphenyl-4-carboxylic acid,
4'-{2-[(1R,2S)-2-hydroxy-2-(4-hydroxyphenyl)-1-methylethylamino]ethoxy}-3'-methyl-3-phenoxybiphenyl-4-carboxylic acid,
4'-{2-[(1R,2S)-2-hydroxy-2-(4-hydroxyphenyl)-1-methylethylamlno]ethoxy}-3-(4-methoxyphenoxy)-3'-methylbiphenyl-4-carboxylic acid,
3'-fluoro-4'-{2-[(1R,2S)-2-hydroxy-2-(4-hydroxyphenyl)-1-methylethylamino]ethoxy}-3-(4-methoxyphenoxy)-biphenyl-4-carboxylic acid,
3-(4-chlorophenoxy)-3'-fluoro-4'-{2-[(1R,2S)-2-hydroxy-2-(4-hydroxyphenyl)-1-methylethylamino]ethoxy}-biphenyl-4-carboxylic acid,
4'-{2-[(1R,2S)-2-hydroxy-2-(4-hydroxyphenyl)-1-methylethylamino]ethoxy}-2'-methyl-3-phenoxybiphenyl-4-carboxylic acid,
3-(4-fluorophenoxy)-4'-{2-[(1R,2S)-2-hydroxy-2-(4-hydroxyphenyl)-1-methylethylamino]ethoxy}-2'-methylbiphenyl-4-carboxylic acid,
4'-{2-[(1R,2S)-2-hydroxy-2-(4-hydroxyphenyl)-1-methylethylamino]ethoxy}-6-methoxy-2'-methylbiphenyl-3-carboxylic acid,
4'-{2-[(1R,2S)-2-hydroxy-2-(4-hydroxyphenyl)-1-methylethylamino]ethoxy}-6-methoxy-3',5'-dimethylbiphenyl-3-carboxylic acid,
6-chloro-4'-{2-[(1R,2S)-2-hydroxy-2-(4-hydroxyphenyl)-1-methylethylamino]ethoxy}-3',5'-dimethylbiphenyl-3-carboxylic acid,
6-chloro-4'-{2-[(1R,2S)-2-hydroxy-2-(4-hydroxyphenyl)-1-methylethylamino]ethoxy}-3'-methylbiphenyl-3-carboxylic acid,
2-ethyl-4'-{2-[(1R,2S)-2-hydroxy-2-(4-hydroxyphenyl)-1-mothylethylamino]ethoxy}biphenyl-4-carboxylic acid,
4'-{2-[(1R,2S)-2-hydroxy-2-(4-hydroxyphenyl)-1-methylethylamino]ethoxy}-2-methylbiphenyl-4-carboxylic acid,
4'-{2-[(1R,2S)-2-hydroxy-2-(4-hydroxyphenyl)-1-methylethylamino]othoxy}-2-isopropylbiphenyl-4-carboxylic acid,
4'-{2-[(1R,2S)-2-hydroxy-2-(4-hydroxyphenyl)-1-methylethylamino]ethoxy}-2-trifluoromethylbiphenyl-4-carboxylic acid,
4'-{2-[(1R,2S)-2-hydroxy-2-(4-hydroxyphenyl)-1-methylethylamino]ethoxy}-3-propylbiphenyl-4-carboxylic acid,
4'-{2-[(1R,2S)-2-hydroxy-2-(4-hydroxyphenyl)-1-methylethylamino]ethoxy}-2-propylbiphenyl-4-carboxylic acid,
3-sec-butyl-4'-{2-[(1R,2S)-2-hydroxy-2-(4-hydroxyphenyl)-1-methylethylamino]ethoxy}biphenyl-4-carboxylic acid,
3-cyclopentyl-4'-{2-[(1R,2S)-2-hydroxy-2-(4-hydroxyphenyl)-1-methylethylamino]ethoxy}biphenyl-4-carboxylic acid,
4'-{2-[(1R,2S)-2-hydroxy-2-(4-hydroxyphenyl)-1-methylethylamino]ethoxy}-3-phenoxybiphenyl-4-carboxylic acid,
4'-{2-[(1R,2S)-2-hydroxy-2-(4-hydroxyphenyl)-1-methylethylamino]ethoxy}-3-(4-methoxyphenoxy)biphenyl-4-carboxylic acid,
3-(4-chlorophenoxy)-4'-{2-[(1R,2S)-2-hydroxy-2-(4-hydroxyphenyl)-1-methylethylamino]ethoxy}biphenyl-4-carboxylic acid,
3-(4-fluorophenoxy)-4'-{2-[(1R,2S)-2-hydroxy-2-(4-hydroxyphenyl)-1-methylethylamino]ethoxy}biphenyl-4-carboxylic acid, or
4'-{2-[(1R,25)-2-hydroxy-2-(4-hydroxyphenyl)-1-methylethylamino]ethoxy}-3-(p-tolyloxy)biphenyl-4-carboxylic acid, or a lower alkyl ester thereof, or a pharmaceutically acceptable salt thereof;

BRL37344, ZD2079, CGP12177, CL316243, L-796568, Ro40-2148, ICID7114, YM-178, solabegron; and β₃ AR stimulants having a β₂ AR stimulating activity as mentioned below and the like can be illustrated.

Among the above compounds, the compounds described in Patent reference 1, especially, 2-[4-[2-[[(1S,2R)-2-hydroxy-2-(4-hydroxyphenyl)-1-methylethyl]amino]ethyl]-2,5-dimethylphenoxy]acetic acid, ethyl 2-[4-[2-[[(1S,2R)-2-hydroxy-2-(4-hydroxyphenyl)-1-methylethyl]amino]ethyl]-2,5-dimethylphenoxy]acetate, or a pharmaceutically acceptable salt thereof are preferable.

A dosage of a β₃ AR stimulant may be determined as needed according to the individual β₃ AR stimulant, body weight, age, sex and degree of diseases of each patient. For example, the range of dosages of a compound described in Patent reference 1 in adults is 0.2 to 200 mg/day in oral administration, 0.0001 to 2% in ocular administration, preferably 0.001 to 0.2%. The compounds described in Patent reference 4 such as Compound 2 as mentioned below or the like can be administered in a similar range of dosage.

A compound having a β₂ AR stimulating activity in addition to a β₃ AR stimulating activity tends to exert more remarkable activities secreting tear and protein in tear than a compound only having a β₃ AR stimulating activity and thus, such a compound is preferable. As a β₃ AR stimulant having a β₂ AR stimulating activity used in the present invention, among the above β₃ AR stimulants, a compound having a stronger β₂ AR stimulating activity than its β₁ AR stimulating activity is preferable, 10 times or stronger one is more preferable, and 100 times or stronger one is further more preferable. As a concrete compound, for example, compounds described in Patent reference 4 can be illustrated. As a preferable compound, for example, compounds described in Patent reference 4: benzyl 2-[3-fluoro-4-[2-[[(1S,2R)-2-hydroxy-2-(4-hydroxyphenyl)-1-methylethyl]amino]ethyl]phenoxy]acetate; 2-[3-chloro-4-[2-[[(1S,2R)-2-hydroxy-2-(4-hydroxyphenyl)-1-methylethyl-amino]ethyl]phenoxy]acetic acid, 2-[4-[2-[[(1S,2R)-2-hydroxy-2-(4-hydroxyphenyl)-1-methylethylamino]ethyl]-phenoxy]acetic acid (hereinafter referred to as Compound 2) or 2-[3-fluoro-4-[2-[[(1S,2R)-2-hydroxy-2-(4-hydroxyphenyl)-1-methylethylamino]ethyl]phenoxy]acetic acid, a lower alkyl ester thereof; or a pharmaceutically acceptable salt thereof and the like can be illustrated. Compound 2 can be easily prepared by a method described in a literature or the like (for example, see Patent reference 4). The activity stimulating each receptor can be determined by methods such as known binding studies, functional studies using extracted organs or the like (for example, see Patent reference 4).

A combination administration of a β₃ AR stimulant and a β₂ AR stimulant can more drastically increase the quantities of tear secretion and protein secretion in tear than a single administration of each stimulant. As a β₂ AR stimulant used in combination with a β₃ AR stimulant in the present invention, a compound having a stronger β₂ AR stimulating activity than its β₁ AR stimulating activity is preferable, 10 times or stronger one is more preferable, and 100 times or stronger one is further more preferable. The activity stimulating each receptor can be determined by methods such as known binding studies, functional studies using extracted organs or the like (for example, see Patent reference 4). As a concrete compound, for example, procaterol, ritodrine, terbutaline, salbutamol, clenbuterol, tulobuterol, mabuterol, salmeterol, formoterol, trimetoquinol, hexoprenaline, methoxyphenamine, orciprenaline, fenoterol and the like and a salt thereof can be illustrated, and especially procaterol or a salt thereof is preferable. These compounds can be commercially available or prepared by a method described in a literature or the like.

In the compounds described in the above Patent references 1 to 3, the term "lower alkyl" means straight or branched alkyl having 1 to 6 carbons, and for example, methyl, ethyl, propyl, isopropyl, butyl, isobutyl, sec-butyl, tert-butyl, pentyl, isopentyl, neopentyl, tert-pentyl, hexyl and the like can be illustrated.

A dosage of a β₂ AR stimulant may be determined as needed according to the individual β₂ AR stimulant or a β₃ AR stimulant combined with, body weight, age, sex and degree of diseases of each patient. For example, the range of dosages of the drug in adults can be 0.001 to 0.2 mg/day of procaterol hydrochloride, 0.01 to 150 mg/day of ritodrine hydrochloride, 0.01 to 15 mg/day of terbutaline sulfate, 0.01 to 15 mg/day of salbutamol sulfate, 0.001 to 0.1 mg/day of clenbuterol hydrochloride, 0.1 to 10 mg/day of tulobuterol hydrochloride, 0.01 to 0.1 mg/day of mabuterol hydrochloride, 0.01 to 0.1 mg/day of salmeterol xinafoate, 0.01 to 0.2 mg/day of formoterol fumarate, 0.1 to 20 mg/day of trimetoquinol hydrochloride, 0.001 to 0.02 mg/day of hexoprenaline, 10 to 300 mg/day of methoxyphenamine hydrochloride, 0.5 to 100 mg/day of orciprenaline sulfate and 0.1 to 10 mg/day of fenoterol hydrobromide in oral administration, and 0.0001 to 1% of procaterol hydrochloride in ocular administration.

A combination pharmaceutical comprising a β₃ AR stimulant and a β₂ AR stimulant of the present invention also includes a single formulation separately comprising the above β₃ AR stimulant and the above β₂ AR stimulant, a formulation in a package that contains both of a formulation containing a β₃ AR stimulant and a formulation containing a β₂ AR stimulant, and a combination of a formulation containing a β₃ AR stimulant and a formulation containing a β₂ AR stimulant that are co-administered simultaneously or at intervals in the same administration form or different administration forms.

A pharmaceutical of the present invention exerts a facilitating activity of tear secretion and protein secretion in tear, and thus, is useful for the prevention or treatment of a disease associated with decrease in tear. In the present invention, the term "disease associated with decrease in tear" means ophthalmic dry symptoms caused qualitative and/or quantitative abnormality and a disorder of the keratoconjunctival epithelium associated therewith and also includes one caused by any causes of decrease in tear secretion and enhanced evaporation or excretion of tear, and, for example, dry eye, dry disorders of cornea and conjunctiva, disorders of the keratoconjunctival epithelium, syndrome with decrease in tear secretion, xerophthalmia, dry eye due to aging, ophthalmopathy in Stevens-Johnson syndrome, ophthalmopathy in Sjögren's syndrome, keratoconjunctival ulcer, oligodacrya, keratoconjunctivitis sicca, ocular pemphigus, blepharitis marginalis, insufficient occlusion of eye lids, sensory neuroparalysis, allergic conjunctivitis, dryness post-viral conjunctivitis, post-cataract surgery, in wearing of contact lens or in operation of visual display terminal (VDT) and the like can be illustrated. Dry eye includes dry eye based on the diagnostic criteria as described in Non-patent reference 1 as well as dry eye diagnosed or suspected based on characteristics such as qualitative or quantitative abnormality (decrease) or disorders of the keratoconjunctival epithelium associated therewith.

In combination pharmaceuticals of the present invention, a formulation comprising a β₃ AR stimulant, a formulation comprising a β₂ AR stimulant, or a single formulation comprising a β₃ AR stimulant and a β₂ AR stimulant can be used as each single formulation or prepared optionally by admixing or by diluting and dissolving a β₃ AR stimulant, a β₂ AR stimulant, or a β₃ AR stimulant and a β₂ AR stimulant with formulation carriers including necessary excipients, disintegrators, binders, lubricants, diluents, buffers, isotonic agents, antiseptics, humectants, emulsifiers, dispersing agents, stabilizers and solubilizers or the like in various dosage forms in the usual way.

Examples of administration forms of the pharmaceutical composition of the present invention are oral formulations such as powders, granules, fine granules, dry syrup, tablets, capsules or the like; parenteral formulations administered non-orally such as eye drops, injections, poultices, suppositories or the like. Oral formulations or eye drops are preferable. Especially, oral formulations are preferable for a patient sensitive to mucosal irritation caused by antiseptics or the like. In case that a formulation comprising a β₃ AR stimulant and a separate formulation comprising a β₂ AR stimulant are administered, these administration forms can be different from each other.

### Examples

The present invention is further illustrated in more detail by way of the following Test examples and Examples. However, the present invention is not limited thereto.

### [Test example 1] Immunostaining test using β₃ AR antibody

The main lacrimal gland was extirpated from male Japanese white rabbits (about 3 kg), soaked and fixed in 10% phosphate buffered formalin, fixed in paraffin and sliced into 3µm sections. After activation of antigenicity, endogenous peroxidase was eliminated, the sections were soaked in the first antibody (β₃ AR antibody) for 24 hrs, rinsed with phosphate buffer (pH 7.4), soaked in the labeled secondary antibody (HRP labeled antibody) for 1 hr and rinsed with a phosphate buffer solution (pH 7.4). After colors were developed with DAB (Diaminobenzidine), the sections were soaked in Mayer-hematoxylin solution for 5 sec, rinsed with running water, dehydrated and cleared for embedding. Microscopic examination confirmed the presence of β₃ AR in rabbit main lacrimal glands (Figure 1).
The immunostaining was performed in the same manner with human lacrimal glands in place of rabbit main lacrimal glands. The presence of β₃ AR was also confirmed in human lacrimal glands (Figure 2).

### [Test example 2] PAS (periodic acid Schiff) staining

The main lacrimal gland was extirpated from male Japanese white rabbits (about 3 kg), soaked and fixed in 10% phosphate buffered formalin, fixed in paraffin and sliced into 3µm sections. After deparaffinization, the sections were rinsed with distilled water, soaked in 1% periodic acid solution for 10 min, rinsed with distilled water, and soaked in Schiff reagent for 10 min. The sections were soaked in 0.5% sodium metabisulfite solution, and the operation was repeated three times. After rinsing with distilled water, the sections were soaked in Mayer-hematoxylin solution for 5 sec, rinsed with running water, dehydrated and cleared for embedding. Microscopic examination confirmed the presence of serous cells and mucous cells in rabbit main lacrimal glands. It was confirmed that the cells on which the presence of β₃ AR was confirmed in Test example 1 were mainly PAS positive cells, that is, mucous cells containing glycoproteins such as mucin (Figure 3).

### [Example 1] Measurement of tear secretion in rabbits given a β₃ AR stimulant

Four fasting male Japanese white rabbits (about 3 kg) were allocated to each group. Compound 1(hydrochloride, 3 or 30 mg/kg), a β₃ AR stimulant, or the vehicle (0.5% gum Arabic) was administered to the duodenum through a needle placed in the duodenum of a rabbit, which was anesthetized with urethane (25%, 5 mL/kg, subcutaneously). The quantity of tear secretion was measured for 5 min before drug administration and for 5 min at 20 min after drug administration in the following manner. One piece each of pre-weighed filter paper (Wattman No. 41, 0.22 mm thick, 2.5 x 1.5 mm) was inserted to each of the upper and lower eyelids of either right or left eye. The difference in weight of the filter papers before and after insertion (post-insertion weight - pre-insertion weight) was defined as the quantity of tear secretion. Fifty (50) µL of a local anesthetic agent, 0.4% oxybuprocaine hydrochloride (Santen Co.), was instilled into eyes 5 min before each measurement. Tear and the instilled local anesthetic were wiped immediately before the filter paper was inserted. Filter papers recovered after each measurement were placed into tubes. A phosphate buffer (pH 7.4) (500 µL) was added to each of the tubes, and they were agitated for 30 sec. After the filter papers were removed and the mixture was centrifuged at 1,880 x g for 5 min, protein concentrations in supernatant were measured using Micro BCA Protein Assay Reagent Kit (Pierce Co.). The quantity of protein in tear was calculated based on the protein concentration and the quantity of tear secretion at each time. The difference in the quantity of tear secretion before administration of a test drug and that at each measurement after administration was defined as a change in the quantity of tear secretion or protein in tear. Each milligram of the change in the quantity of tear was taken as 1 µL in tabulation. As a result, administration of Compound 1 (hydrochloride, 3 or 30 mg/kg) into the duodenum facilitated the tear secretion and protein secretion in tear. Administration of Compound 1 (30 mg/kg) significantly increased the change in the quantity of protein in tear in rabbits compared to Control group (Figure 4).

In addition, in order to study the role played by a β₂ AR stimulating activity in the above-described actions of Compound 1, 30 µg/kg of ICI-118551 (Sigma Co.), a selective β₂ AR inhibitor, was administered intravenously 5 min before administration of Compound 1 (30 mg/kg) and the same test was performed. The facilitating activities of tear secretion and protein secretion in tear by Compound 1 were not affected by pre-treatment with the selective β₂ AR inhibitor, ICI-118551 (30 µg/kg, i.v.) (Figure 5). The results confirmed that the Compound 1's activities facilitating tear secretion and protein secretion in tear were exerted by way of β₃ AR.

### [Example 2] Measurement of tear in rabbits given a β₃ AR stimulant having a β₂ AR stimulating activity

Four fasting male Japanese white rabbits (about 3 kg) were allocated to each group. Compound 2 (0.3, 1 or 10 mg/kg), which is a β₃ AR stimulant having a β₂ AR stimulating activity, terbutaline sulfate (10 mg/kg), which is a β₂ AR stimulant, or their vehicle (distilled water) was administered to the duodenum through a needle placed in the duodenum of a rabbit which was anesthetized with urethane (25%, 5 mL/kg, subcutaneously). In the same manner as in Example 1, quantities of tear secretion and protein in tear were measured for each 5 min before drug administration and at 5, 20, 30, 40 and 50 min after drug administration. The total quantities of tear secretion and protein in tear were defined as the sum total of the quantities of secretion at each measurement time during 60 min after administration of the test drug. Each gram of the change in the quantity of tear was considered as 1 µL in tabulation.

The results of measurement of the total quantities of tear secretion and protein in tear are shown in Figure 6. Administration of Compound 2 (0.3, 1 and 10 mg/kg) into the duodenum facilitated dose-dependently the tear secretion and protein secretion in tear in rabbits, and, at doses of 1 mg/kg and above, significantly increased the total quantities of tear secretion and protein secretion in tear compared to Control group. On the other hand, administration of terbutaline sulfate (10 mg/kg) into the duodenum significantly increased the total quantities of tear secretion and protein secretion in tear compared to Control group. However, the increases by terbutaline sulfate were significantly lower than those in Compound 2 (10 mg/kg) group.

### [Example 3] Measurement of tear in rats given a β₃ AR stimulant having a β₂ AR stimulating activity

Nine (9) to 10 fasting male SD strain rats (7 weeks old) were allocated to each group. Rats were fixed in prone position under urethane anesthesia (25%, 5 mL/kg, subcutaneously). Compound 2 (0.3, 1 or 10 mg/kg), which is a β₃ AR stimulant having a β₂ AR stimulating activity, terbutaline sulfate (10 mg/kg), which is a β₂ AR stimulant, or their vehicle (distilled water) were administered to the duodenum through a needle placed in the duodenum. One end of a capillary (Drummond Microdispenser Co. 10 µL) was placed at the inner canthus of rat right eye after tear was wiped. The quantity of tear secretion was measured by the length of the capillary filled with tear in 60 minutes after drug administration. The total quantity of tear secretion (µL) was calculated using the inner diameter of the capillary and the length of the capillary filled with tear. After the quantity of tear secretion was measured, the tear in the capillary was recovered in a tube to measure the protein concentration using Micro BCA Protein Assay Reagent Kit (Pierce Co.). The total quantity of protein in tear was calculated using the obtained protein concentration and the total quantity of tear secretion.

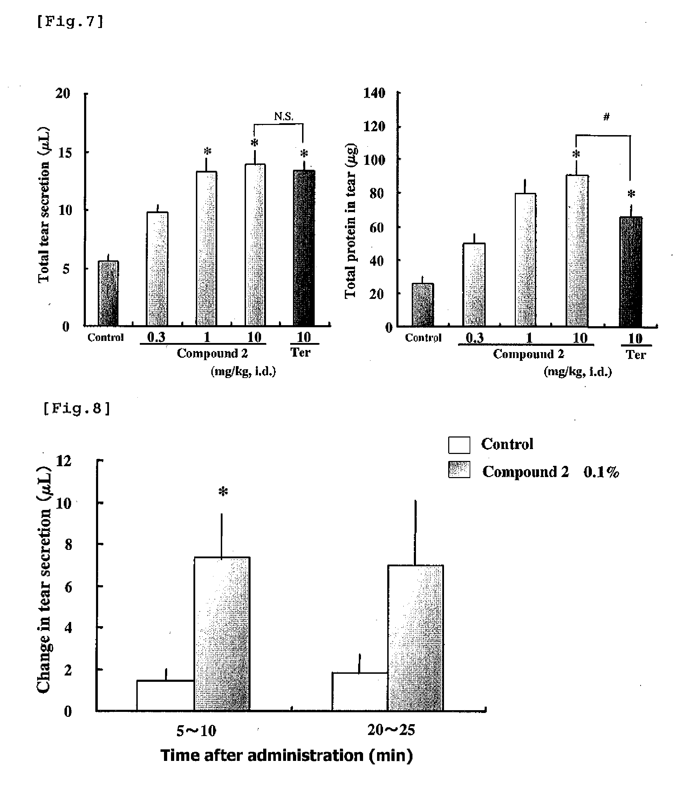

The results of measurement of the total quantities of tear secretion and protein in tear are shown in Figure 7. Administration of Compound 2 (0.3, 1 and 10 mg/kg) into the duodenum facilitated dose-dependently the tear secretion and protein secretion in tear in rats, and, at doses of 1 mg/kg and above, significantly increased the total quantities of tear secretion and protein secretion in tear compared to Control group. On the other hand, administration of terbutaline sulfate (10 mg/kg) into the duodenum significantly increased the total quantities of tear secretion and protein secretion in tear compared to Control group. However, the total quantity of protein in tear induced by terbutaline sulfate was significantly lower than that in Compound 2 (10 mg/kg) group.

### [Example 4] Measurement of tear in rabbits following ocular instillation of a β₃ AR stimulant having a β₂ AR stimulating activity

Four fasting male Japanese white rabbits (about 3 kg) were allocated to each group. After rabbits were anesthetized with urethane (25%, 5 mL/kg, subcutaneously), 50 µL of Compound 2 (0.1% solution) or the vehicle (a phosphate buffer solution, pH 7.4) was instilled in either right or left eye. The quantity of tear secretion was measured and calculated for each 5 min before and at 5 and 20 min after instillation in the same manner as in Example 1. The difference in the quantity of tear secretion before administration of a test drug and that at each measurement time after ocular instillation was defined as a change in the quantity of tear secretion. Each milligram of the change in the quantity of tear secretion was considered as 1 µL in tabulation. As a result, ocular instillation of Compound 2 (0.1% solution) facilitated tear secretion in rabbits and significantly increased the quantity of tear secretion measured for 5 min after ocular instillation compared to Control group (Figure 8).

### [Example 5] Mucin secretion from the rabbit conjunctiva following ocular instillation of a β₃ AR stimulant having a β₂ AR stimulating activity (impression cytology method)

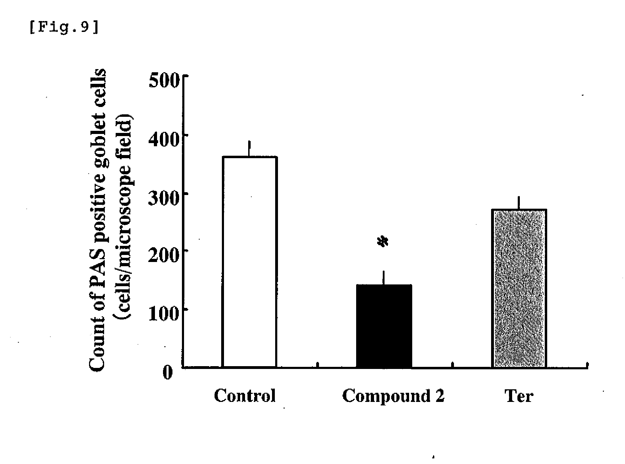

Four male Japanese white rabbits (about 3 kg) were allocated to each group. After rabbits were anesthetized with ketamine-xylazine (3:1), 50 µL of Compound 2 (0.1% solution), terbutaline sulfate (0.1% solution) or their vehicle (a phosphate buffer solution, pH 7.4) were instilled in either side of eyes. Mucin secretion from the conjunctiva was measured 30 min after instillation by impression cytology method. That is, after ocular instillation of 50 µL of a local anesthetic (0.4% oxybuprocaine hydrochloride by Santen Co.), residual tear was wiped 5 min after instillation and 3 x 3 mm cellulose acetate filter paper was pressed on the conjunctiva for 30 sec. Then, PAS staining was performed with the recovered filter papers in the same manner as in Example 2. After filter papers were cleared and embedded, the number of PAS positive goblet cells existing in a certain visual field of a microscope was counted. Decreases in the count of PAS positive goblet cells indicate that mucin secretion from the conjunctiva is facilitated. As a result, Compound 2 facilitated mucin secretion from the conjunctiva more than the vehicle did. However, terbutaline sulfate had no effect (Figure 9).

As mentioned above, a β₃ AR stimulant dose-dependently increased the quantities of tear secretion and protein secretion in tear in rats or rabbits, especially, the quantity of protein in tear and also increased the quantity of mucin secretion from the conjunctiva. In addition, a β₃ AR stimulant in combination with a β₂ AR stimulant exerted a significantly increasing effect compared to a β₂ AR stimulant. Such a result was observed in both of intraduodenal and ocular administration. Thus, a β₃ AR stimulant exerted a remarkably increasing effect of the quantities of tear secretion and protein secretion in tear by a single use or a combination use with a β₂ AR stimulant.

### Industrial applicability

The pharmaceuticals of the present invention are extremely useful as agents for the prevention or treatment of diseases associated with decrease in tear.

## Claims

1. A pharmaceutical for the prevention or treatment of a disease associated with decrease in tear, which comprises a β₃-adrenoceptor stimulant.

2. A pharmaceutical for the facilitation of tear secretion, which comprises a β₃-adrenoceptor stimulant.

3. A pharmaceutical for the facilitation of protein secretion in tear, which comprises a β₃-adrenoceptor stimulant.

4. A pharmaceutical as claimed in any of claims 1 to 3 wherein the β₃-adrenoceptor stimulant is 2-[4-[2-[[(1S, 2R)-2-hydroxy-2-(4-hydroxyphenyl)-1-methylethyl]amino]-ethyl]-2,5-dimethylphenoxy]acetic acid, 2-[2-bromo-4-[2-[[(1S,2R)-2-hydroxy-2-(4-hydroxyphenyl)-1-methylethyl]-amino]ethyl]phenoxy]acetic acid, 2-[2-chloro-4-[2-[[(1S, 2R)-2-hydroxy-2-(4-hydroxyphenyl)-1-methylethyl]amino]-ethyl]phenoxy]acetic acid, 2-[2,5-dichloro-4-[2-[[(1S,2R)-2-hydroxy-2-(4-hydroxyphenyl)-1-methylethyl]amino]ethyl]-phendxy]acetic acid, 2-[4-[2-[[(1S,2R)-2-hydroxy-2-(4-hydroxyphenyl)-1-methylethyl]amino]ethyl]-2,5-dimethyl-phenoxy]acetic acid or 2-[2-hydroxy-4-[2-[[(1S,2R)-2-hydroxy-2-(4-hydroxyphenyl)-1-methylethyl]amino]-ethyl]phenoxy]acetic acid, or a lower alkyl ester thereof; or a pharmaceutically acceptable salt thereof.

5. A pharmaceutical as claimed in any of claims 1 to 3 wherein the β₃-adrenoceptor stimulant is 4'-{2-[(1S,2R)-2-hydroxy-2-(4-hydroxyphenyl)-1-methylethylamino]ethoxy}-2,3',5'-trimethylbiphenyl-4-carboxylic acid, 4'-{2-[(1S, 2R)-2-hydroxy-2-(4-hydroxyphenyl)-1-methylethylamino]-ethoxy}-3-isopropyl-3',5'-dimethylbiphenyl-4-carboxylic acid, (3-acetyl-4'-{2-[(1S,2R)-2-hydroxy-2-(4-hydroxyphenyl)-1-methylethylamino]ethoxy}-3',5'-dimethylbiphenyl-4-yloxy)acetic acid, 4'-{2-[(1R,2S)-2-hydroxy-2-(4-hydroxyphenyl)-1-methylethylamino]ethoxy}-2,2'-dimethylbiphenyl-4-caboxylic acid, 2-ethyl-4'-{2-[(1S,2R)-2-hydroxy-2-(4-hydroxyphenyl)-1-methylethylamino]ethoxy}-2'-methylbiphenyl-4-carboxylic acid, 4'-{2-[(1S,2R)-2-hydroxy-2-(4-hydroxyphenyl)-1-methylethylamino]ethoxy}-2-isopropyl-2'-methylbiphenyl-4-carboxylic acid, 4'-{2-[(1S,2R)-2-hydroxy-2-(4-hydroxyphenyl)-1-methylethylamino]ethoxy}-2'-methyl-2-propylbiphenyl-4-carboxylic acid, 4'-{2-[(1R,2S)-2-hydroxy-2-(4-hydroxyphenyl)-1-methylethylamino]ethoxy}-2-methoxy-3',5'-dimethylbiphenyl-4-carboxylic acid, 4'-{2-[(1R,2S)-2-hydroxy-2-(4-hydroxyphenyl)-1-methylethyl-amino]ethoxy}-3',5'-dimethyl-2-propylbiphenyl-4-carboxylic acid, 2-ethyl-4'-{2-[(1R,2S)-2-hydroxy-2-(4-hydroxyphenyl)-1-methylethylamino]ethoxy}-3'-methylbiphenyl-4-carboxylic acid, 4'-{2-[(1R,2S)-2-hydroxy-2-(4-hydroxyphenyl)-1-methylethylamino]ethoxy}-3'-methyl-2-propylbiphenyl-4-carboxylic acid, 3-cyclopentyl-4'-{2-[(1R,2S)-2-hydroxy-2-(4-hydroxyphenyl)-1-methylethylamino]ethoxy}-3'-methylbiphenyl-4-carboxylic acid, 2-ethyl-3'-fluoro-4'-{2-[(1R,2S)-2-hydroxy-2-(4-hydroxyphenyl)-1-methylethylamino]-ethoxy}biphenyl-4-carboxylic acid, 3'-fluoro-4'-{2-[(1R,2S)-2-hydroxy-2-(4-hydroxyphenyl)-1-methylethylamino]-ethoxy}-2-isopropylbiphenyl-4-carboxylic acid, 3'-fluoro-4'-{2-[(1R,2S)-2-hydroxy-2-(4-hydroxyphenyl)-1-methylethyl-amino]ethoxy}-2-propylbiphenyl-4-carboxylic acid, {4'-{2-[(1S,2R)-2-hydroxy-2-(4-hydroxyphenyl)-1-methylethylamino]-ethoxy}-2,3',5'-trimethylbiphenyl-4-yloxy)acetic acid, 3-hyroxy-4'-(2-[(1S,2R)-2-hydroxy-2-(4-hydroxyphonyl)-1-methylethylamino]ethoxy}-3',5'-dimethylbiphenyl-4-carboxylic acid, 4'-{2-[(1R,2S)-2-hydroxy-2-(4-hydroxyphenyl)-1-methylethylamino]ethoxy}-3',5'-dimethyl-3-(p-tolyloxy)biphenyl-4-carboxylic acid, 3-(4-chlorophenoxy)-4'-{2-[(1R,2S)-2-hydroxy-2-(4-hydroxyphenyl)-1-methylethyl-amino]ethoxy}-3',5'-dimethylbiphenyl-4-carboxylic acid, 3-(4-fluorophenoxy)-4'-{2-[(1R,2S)-2-hydroxy-2-(4-hydroxyphenyl)-1-methylethylamino]ethoxy}-3',5'-dimethylbiphenyl-4-carboxylic acid, 4'-{2-[(1R,2S)-2-hydroxy-2-(4-hydroxyphenyl)-1-methylethylamino]ethoxy}-3-(4-methoxyphenoxy)-3' ,5'-dimethylbiphenyl-4-carboxylic acid, 4'-{2-[(1R,2S)-2-hydroxy-2-(4-hydroxyphenyl)-1-methylethylamino]ethoxy}-3'-methyl-3-phenoxybiphenyl-4-carboxylic acid, 4'-{2-[(1R,2S)-2-hydroxy-2-(4-hydroxyphenyl)-1-methylethylamino]ethoxy}-3-(4-methoxyphenoxy)-3'-methylbiphenyl-4-carboxylic acid, 3'-fluoro-4'-{2-[(1R,2S)-2-hydroxy-2-(4-hydroxyphenyl)-1-methylethylamino]ethoxy}-3-(4-methoxyphenoxy)biphenyl-4-carboxylic acid, 3-(4-chlorophenoxy)-3'-fluoro-4'-{2-[(1R,2S)-2-hydroxy-2-(4-hydroxyphenyl)-1-methylethyl-amino]ethoxy}biphenyl-4-carboxylic acid, 4'-{2-[(1R,2S)-2-hydroxy-2-(4-hydroxyphenyl)-1-methylethylamino]ethoxy}-2'-methyl-3-phenoxybiphenyl-4-carboxylic acid, 3-(4-fluoro-phenoxy)-4'-{2-[(1R,2S)-2-hydroxy-2-(4-hydroxyphenyl)-1-methylethylamino]ethoxy}-2'-methylbiphenyl-4-carboxylic acid, 4'-{2-[(1R,2S)-2-hydroxy-2-(4-hydroxyphenyl)-1-methylethylamino]ethoxy}-6-methoxy-2'-methylbiphenyl-3-carboxylic acid, 4'-{2-[(1R,2S)-2-hydroxy-2-(4-hydroxyphenyl)-1-methylethylamino]ethoxy}-6-methoxy-3',5'-dimethylbiphenyl-3-carboxylic acid, 6-chloro-4'-{2-[(1R,2S)-2-hydroxy-2-(4-hydroxyphenyl)-1-methylethyl-amino]ethoxy}-3',5'-dimethylbiphenyl-3-carboxylic acid, 6-chloro-4'-{2-[(1R,2S)-2-hydroxy-2-(4-hydroxyphenyl)-1-methylethylamino]ethoxy}-3'-methylbiphenyl-3-carboxylic acid, 2-ethyl-4'-{2-[(1R,2S)-2-hydroxy-2-(4-hydroxyphenyl)-1-methylethylamino]ethoxy}biphanyl-4-carboxylic acid, 4'-{2-[(1R,2S)-2-hydroxy-2-(4-hydroxyphenyl)-1-methylethyl-amino]ethoxy}-2-methylbiphenyl-4-carboxylic acid, 4'-{2-[(1R,2S)-2-hydroxy-2-(4-hydroxyphenyl)-1-methylethyl-amino]ethoxy}-2-isopropylbiphenyl-4-carboxylic acid, 4'-{2-[(1R,2S)-2-hydroxy-2-(4-hydroxyphenyl)-1-methylethyl-amina]ethoxy}-2-trifluoromethylbiphenyl-4-carboxylic acid, 4'-{2-[(1R,2S)-2-hydroxy-2-(4-hydroxyphenyl)-1-methylethyl-amino]ethoxy}-3-propylbiphenyl-4-carboxylic acid, 4'-{2-[(1R,2S)-2-hydroxy-2-(4-hydroxyphenyl)-1-methylethyl-amino]ethoxy}-2-propylbiphenyl-4-carboxylic acid,3-sec-butyl-4'-{2-[(1R,2S)-2-hydroxy-2-(4-hydroxy-phenyl)-1-methylethylamino]ethoxy}biphanyl-4-carboxylic acid, 3-cyclopentyl-4'-{2-[(1R,2S)-2-hydroxy-2-(4-hydroxyphenyl)-1-methylethylamino]ethoxy}biphenyl-4-carboxylic acid, 4'-{2-[(1R,2S)-2-hydroxy-2-(4-hydroxyphenyl)-1-methyl-ethylamino]ethoxy}-3-phenoxybiphenyl-4-carboxylic acid, 4'-{2-[(1R,2S)-2-hydroxy-2-(4-hydroxyphenyl)-1-methylethyl-amino]ethoxy}-3-(4-methoxyphenoxy)biphenyl-4-carboxylic acid, 3-(4-chlorophenoxy)-4'-{2-[(1R,2S)-2-hydroxy-2-(4-hydroxyphenyl)-1-methylethylamino]ethoxy}biphenyl-4-carboxylic acid, 3-(4-fluorophenoxy)-4'-{2-[(1R,2S)-2-hydroxy-2-(4-hydroxyphenyl)-1-methylethylamino]ethoxy}-biphenyl-4-carboxylic acid or 4'-{2-[(1R,2S)-2-hydroxy-2-(4-hydroxyphenyl)-1-methylethylamino]ethoxy}-3-(p-tolyloxy)biphenyl-4-carboxylic acid, or a lower alkyl ester thereof, or a pharmaceutically acceptable salt thereof.

6. A pharmaceutical as claimed in any of claims 1 to 3 wherein the β₃-adrenoceptor stimulant is BRL37344, ZD2079, CGP12177, CL316243, L-796568, Ro40-2148, ICID7114, YM-178 or solabegron, or a pharmaceutically acceptable salt thereof.

7. A pharmaceutical as claimed in any of claims 1 to 3 wherein the β₃-adrenoceptor stimulant is a β₃-adrenoceptor stimulant having a β₂-adrenoceptor stimulating activity.

8. A pharmaceutical as claimed in claim 7 wherein the β₃-adrenoceptor stimulant having a β₂-adrenoceptor stimulating activity is benzyl 2-[3-fluoro-4-[2-[[(1S,2R)-2-hydroxy-2-(4-hydroxyphenyl)-1-methylethyl-amino]ethyl]phenoxy]acetate; or 2-[3-chloro-4-[2-[[(1S,2R)-2-hydxoxy-2-(4-hydroxyphenyl)-1-methylethylamino]ethyl]-phenoxy]acetic acid, 2-[4-[2-[[(1S,2R)-2-hydroxy-2-(4-hydroxyphenyl)-1-methylethylamino]ethyl]phenoxy]acetic acid or 2-[3-fluoro-4-[2-[[(1S,2R)-2-hydroxy-2-(4-hydroxyphenyl)-1-methylethylamino]ethyl]phenoxy]acetic acid, or a lower alkyl ester thereof; or a pharmaceutically acceptable salt thereof.

9. A pharmaceutical as claimed in any of claims 1 to 6 which is a combination additionally comprising a β₂ adrenoceptor stimulant.

10. A pharmaceutical as claimed in claim 9 wherein the β₂ adrenoceptor stimulant is procaterol, ritodrine, terbutaline, salbutamol, clenbuterol, tulobuterol, mabuterol, salmeterol, formoterol, trimetoquinol, hexoprenaline, methoxyphenamine, orciprenaline or fenoterol, or a pharmaceutically acceptable salt thereof.

11. A pharmaceutical as claimed in any of claims 1 and 4 to 10 wherein the disease associated with decrease in tear are one or more diseases selected from the group consisting of dry eye, dry disorders of cornea and conjunctiva, disorders of the keratoconjunctival epithelium, syndrome with decrease in tear secretion, xerophthalmia, dry eye due to aging, ophthalmopathy in Stevens-Johnson syndrome, ophthalmopathy in Sjögren's syndrome, keratoconjunctival ulcer, oligodacrya, keratoconjunctivitis sicca, ocular pemphigus, blepharitis marginalis, insufficient occlusion of eye lids, sensory neuroparalysis, allergic conjunctivitis, and dryness post-viral conjunctivitis, post-cataract surgery, in wearing of contact lens or in operation of visual display terminal (VDT).

12. A pharmaceutical as claimed in any of claims 1 to 11 wherein the dosage form is an oral formulation.

13. A pharmaceutical as claimed in any of claims 1 to 11 wherein the dosage form is a parenteral formulation.

14. A pharmaceutical as claimed in claim 12 wherein the parenteral formulation is an eye drop.
